# EUROPEAN PATENT SPECIFICATION

(11) **EP 3 982 972 B1**
(45) Date of publication and mention of the grant of the patent: **12.08.2026**
(21) Application number: 20822711.6
(22) Date of filing: 08.06.2020
(51) Int. Cl.: A61K 31/5383, A61K 31/519, A61K 31/175, A61K 31/426, A61K 45/06, A61P 1/12

(54) **BPO-27 FOR TREATING BILE ACID DIARRHEA**
BPO-27 ZUR BEHANDLUNG VON GALLENSÄUREDIARRHOE
BPO-27 POUR LE TRAITEMENT DE LA DIARRHÉE À ACIDES BILIAIRES

(30) Priority: 12.06.2019 US 201962860539 P
(43) Date of publication of application: 20.04.2022
(73) Proprietor: The Regents of the University of California, Oakland, CA 94607-5200 (US)
(72) Inventor: VERKMAN, Alan S., Oakland, California 94607-5200 (US)
(74) Representative: HGF
(86) International application number: PCT/US2020/036683
(87) International publication number: WO 2020/251906

(56) References cited:
- WO-A1-2018/222921
- US-A1- 2007 254 050
- US-A1- 2014 080 821
- US-A1- 2017 224 721
- ZHANG WEIQIANG ET AL: "Recent advances and new perspectives in targeting CFTR for therapy of cystic fibrosis and enterotoxin-induced secretory diarrheas", FUTURE MEDICINAL CHEMISTRY, vol. 4, no. 3, 1 March 2012 (2012-03-01), GB, pages 329 - 345, XP093043502, ISSN: 1756-8919, DOI: 10.4155/fmc.12.1
- VERKMAN ALAN S ET AL: "CFTR Inhibitors", CURRENT PHARMACEUTICAL DESIGN, 2913, 19, 1 January 2013 (2013-01-01), pages 3529 - 3541, XP093030660, Retrieved from the Internet <URL:https://www.ncbi.nlm.nih.gov/pmc/articles/PMC3320517/pdf/nihms364141.pdf> [retrieved on 20230310]
- DUAN ET AL.: "Inhibition of CFTR-mediated intestinal chloride secretion as potential therapy for bile acid diarrhea", THE FASEB JOURNAL, vol. 33, October 2019 (2019-10-01), pages 10924 - 10934, XP055772270, DOI: https://faseb.onlinelibrary.wiley.com/doi/full/10.1096/fj.201901166R

## Description

### GOVERNMENT RIGHTS

This invention was made with Government support under Grant Nos. DK099803, DK072517, DK101373, EY013574 and DK089502 awarded by The National Institutes of Health.

### TECHNICAL FIELD

The present invention is directed to methods of treating bile acid diarrhea by administering a CFTR chloride channel inhibitor.

### BACKGROUND

Bile acid diarrhea (BAD) is commonly seen following ileal resection and in Crohn's disease, and is believed to occur in more than one-third of patients with diarrhea-predominant irritable bowel syndrome (IBS-D) or chronic functional diarrhea, with an estimated overall prevalence of up to 1% in Western countries. BAD can be caused by abnormalities in hepatic bile acid synthesis or enterohepatic circulation, which results in the delivery of excess bile acids to the colon where they cause fluid secretion and increase motility.

Current therapy for primary BAD, or BAD caused by ileal resection or Crohn's disease, includes bile acid binders such as cholestyramine, colestipol and colesevelam, and farnesoid X receptor (FXR) agonists such as obeticholic acid are in development. For IBS-D, in which BAD is likely involved in pathogenesis in more than one-third of patients, FDA-approved therapies include the 5-HT3 antagonist alosetron, the mixed µ opioid receptor agonist eluxadoline and the broad-spectrum gut-specific antibiotic rifaximin. Other commonly used therapies for IBS-D include loperamide, bile acid sequestrants, antispasmodics and tricyclic antidepressants. Some of these therapies are associated with significant side effects such as ischemic colitis with alosetron and pancreatitis with eluxadoline.

An unmet need for improved and alternative therapeutics for BAD persists.

The Cystic Fibrosis Transmembrane Conductance Regulator (CFTR) protein is a chloride channel expressed in certain mammalian epithelial cells, including intestinal epithelial cells. CFTR chloride channel function has been associated with secretory diarrhea.

CFTR inhibitors have clinical application in the therapy of secretory diarrheas. Secretory diarrheas caused by enterotoxins, such as cholera and Travelers' diarrhea (enteropathogenic E. coli), require functional CFTR for primary chloride secretion into the intestinal lumen, which secondarily drives sodium and water secretion (see, e.g., Kunzelmann et al., Physiol. Rev. 82:245-89 (2002); Thiagarajah et al., Curr. Opin. Pharmacol. 3:594-9 (2003)). Cell culture and animal models indicated that intestinal chloride secretion in enterotoxin-mediated secretory diarrheas occurs mainly through CFTR (see, e.g., Clarke et al., Science 257: 1125-28 (1992); Gabriel et al., Science 266:107-109 (1994); KunzelmannandMall,Physiol. Rev. 82:245-89 (2002); Field, 15 J. Clin. Invest. 111:931-43 (2003); and Thiagarajah et al., Gastroenterology 126:511-519 (2003)). Several classes of small molecule CFTR inhibitors have been described previously (see, e.g., review by Verkman et al., Nat. Rev. Drug Discov. 8:153-71 (2009)).

U.S. Patent No. 9,062,073 describes a series of benzopyrimido-pyrrolo-oxazine-dione (BPO) compounds and certain pyrimido-pyrrolo-quinoxalinedione (PPQ) compounds which are useful as CFTR chloride channel inhibitors. The BPO compounds have the generic formula: 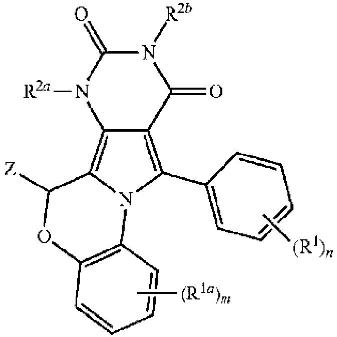 wherein:
m is 1, 2, 3, or 4;
n is 1, 2, 3, 4 or 5;
p is an integer from 0 to 4;
q is an integer from 1 to 4;
R¹ at each occurrence is the same or different and independently H, halo, haloalkyl, C₁-C₆ alkyl, -(CH₂)ₚ-C(O)-R^{4a}, -S(O)₂R^{4a}, -NO₂, or tetrazolyl;
R^{1a} at each occurrence is the same or different and independently H, halo, haloalkyl, C₁-C₆ alkyl, -(CH₂)ₚ-C(O)-R^{4a}, -S(O)₂R^{4a}, -NO₂, or tetrazolyl;
R^{2a} and R^{2b} are each the same or different and independently H, or C₁-C₆ alkyl;
R^{4a} is -OR⁷, -NR⁷R⁸ -O(CH₂)_{q-}OC(O)R⁷, or an amino acid residue;
R⁷ and R⁸ are each the same or different and independently H, C₁-C₂₀ alkyl, a saccharide, or an amino acid residue; and
Z is aryl or heteroaryl,
wherein the amino acid residue is selected from residues of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, phosphoserine, phosphothreonine, phosphotyrosine, 4-hydroxyproline, hydroxylysine, demosine, isodemosine, gamma-carboxy glutamate, hippuric acid, octahydroindole-2-carboxylic acid, statine, 1,2,3,4-tetrahydroisoquinoline-3-carboxylic acid, penicillamine, ornithine, 3-methylhistidine, norvaline, beta-alanine, gamma-aminobutylic acid, cirtulline, homocysteine, homoserine, methyl-alanine, para-benzoylphenylalanine, phenylglycine, propargylglycine, sarcosine, methionine sulfone, tert-butylglycine, 3,5-dibromotyrosine, 3,5-diiodotyrosine, glycosylated threonine, glyclosylated serine, and glycosylated asparagine.

The PPQ compounds have the generic formula: 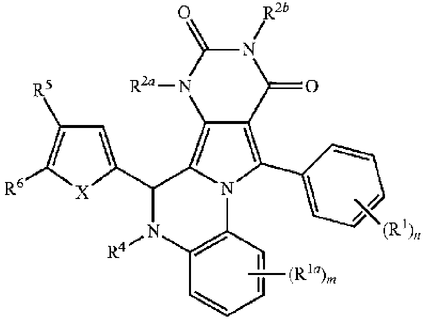 wherein:
m is 1, 2, 3, or 4;
n is 1, 2, 3, 4 or 5;
p is an integer from 0 to 4;
q is an integer from 1 to 4;
X is O or S;
R¹ at each occurrence is the same or different and independently H, halo, haloalkyl, C₁₋₆ alkyl, -(CH₂)ₚ-C(O)-R^{4a}, -S(O)₂R^{4a}, -NO₂, or tetrazolyl;
R^{1a} at each occurrence is the same or different and independently H, halo, haloalkyl, C₁₋₆ alkyl, -(CH₂)ₚ-C(O)-R^{4a}, -S(O)₂R^{4a}, -NO₂, or tetrazolyl;
R^{2a} and R^{2b} are each the same or different and independently H or C₁₋₆ alkyl;
R^{4a} is -OR⁷, -NR⁷R⁸ -O(CH₂)_{q-}OC(O)R⁷, an amino acid residue, or a peptide;
R⁴ is H, -N(=O), C₁₋₆ alkyl, or haloalkyl;
R⁵ is H, halo, or C₁₋₆ alkyl;
R⁶ is halo, C₁₋₆ alkyl, or C₁₋₆ haloalkyl; and
R⁷ and R⁸ are each the same or different and independently H, C₁₋₂₀ alkyl, a saccharide, an amino acid residue, or a peptide.

WO 2018/222921 discloses methods for treating bile acid diarrhea, short bowel syndrome (SBS), and cholecystectomy-associated diarrhea by administering to a patient in need thereof, an inhibitor of chloride-ion transport in an amount sufficient to treat diarrhea. The disclosed treatment of diarrhea includes the treatment of the diarrhea as well as the pain, abdominal discomfort and other symptoms associated with diarrhea. It is disclosed that the inhibitor of chloride-ion transport may be crofelemer. WO 2018/222921 discloses only two individual patient cases - one with short bowel syndrome and another with post-cholecystectomy diarrhea. In both cases, while improvement with Crofelemer is reported, bile acid diarrhea is not confirmed.

### SUMMARY

The present invention is directed to BPO-27, or a pharmaceutical composition comprising BPO-27, for use in a method of treating a subject having bile acid diarrhea.

In addition, aspects of the present invention are also directed to methods of reducing intestinal fluid secretion resulting from bile acid-induced activation of apical CFTR chloride channels in the intestinal epithelium in a subject in need thereof, comprising administering to the subject an amount of a CFTR chloride channel inhibitor, i.e., BPO-27, effective to reduce said intestinal fluid secretion resulting from bile acid-induced activation of apical CFTR chloride channels.

Other aspects of the invention are directed to methods of reducing intestinal fluid secretion resulting from bile acid-induced activation of apical CFTR chloride channels in the intestinal epithelium in a subject in need thereof, comprising administering to the subject a pharmaceutical composition comprising an amount of a CFTR chloride channel inhibitor, i.e., BPO-27, effective to reduce said intestinal fluid secretion resulting from bile acid-induced activation of apical CFTR chloride channels.

Further aspects of the present invention are directed to methods of reducing bile acid-induced apical CFTR chloride channel current in the intestinal epithelium of a subject in need thereof, comprising administering to the subject an amount of a CFTR chloride channel inhibitor, i.e., BPO-27, effective to reduce said bile acid-induced apical CFTR chloride channel current.

Other aspects of the invention are directed to methods of reducing bile acid-induced apical CFTR chloride channel current in the intestinal epithelium of a subject in need thereof, comprising administering to the subject a pharmaceutical composition comprising an amount of a CFTR chloride channel inhibitor, i.e., BPO-27, effective to reduce said bile acid-induced apical CFTR chloride channel current.

In some aspects, not forming part of the present invention, the disclosure is directed to methods for treating diarrhea, or alleviating symptoms associated with diarrhea, in a subject who has undergone ileal resection, comprising administering to the subject an amount of a CFTR chloride channel inhibitor effective to treat said diarrhea or to alleviate the symptoms of said diarrhea.

Other aspects, not forming part of the present invention, the disclosure are directed to methods for treating diarrhea, or alleviating symptoms associated with diarrhea, in a subject who has undergone ileal resection, comprising administering to the subject a pharmaceutical composition comprising an amount of a CFTR chloride channel effective to treat said diarrhea or to alleviate the symptoms of said diarrhea.

The references to methods of treatment in this description are to be interpreted as references to the compounds, pharmaceutical compositions and medicaments of the present invention for use in a method for treatment of the human (or animal) body by therapy.

### BRIEF DESCRIPTION OF THE DRAWINGS

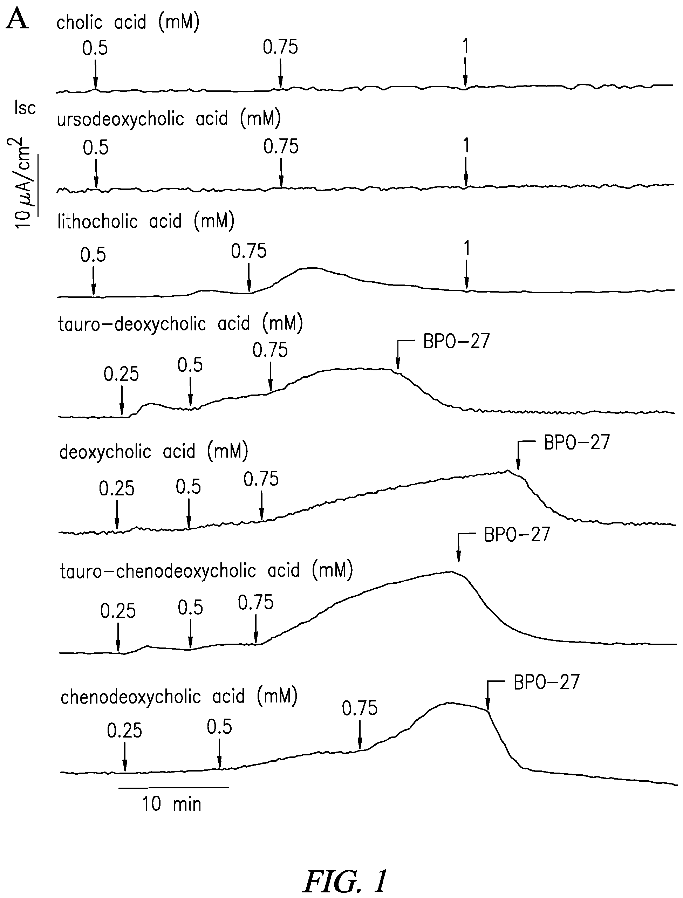

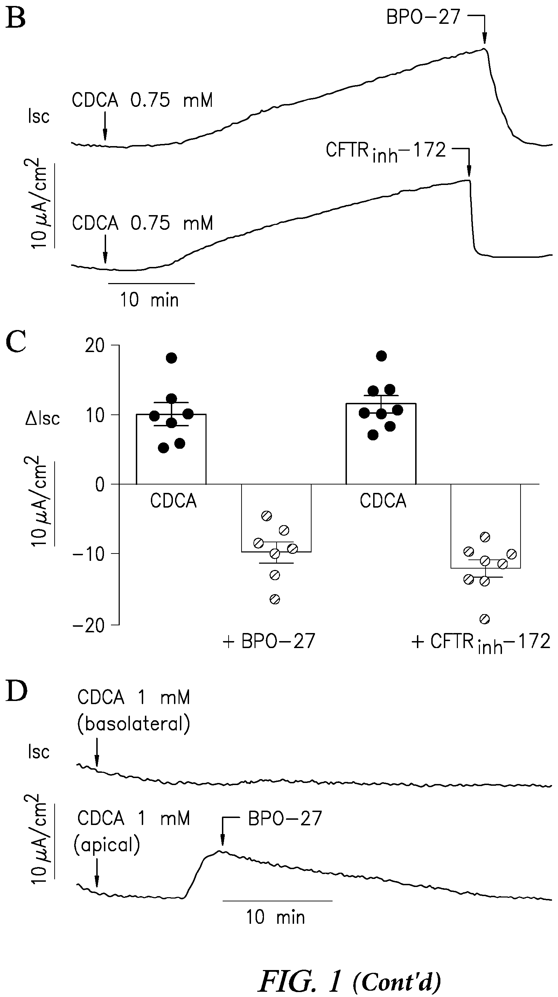

**Figure 1****.** CFTR inhibitors block bile acid-induced secretory current in T84 cells. **(A.)** Short-circuit current (Isc) showing the response to indicated bile acids added to both apical and basolateral bathing solutions. (R)-BPO-27 is added at 10 µM (representative of 3 filters studied for cholic acid, ursodeoxycholic acid and lithocholic acid; and 5 filters for deoxycholic acid, chenodeoxycholic acid and their tauro-conjugates). **(B.)** CDCA (0.75 mM) is added to both bathing solution, with (R)-BPO-27 (10 µM) or CFTRᵢₙₕ-172 (10 µM) added as indicated. **(C.)** Summary of peak CDCA-induced current and the reduction in current after (R)-BPO-27 or CFTRᵢₙₕ-172 (ΔIsc, mean ± S.E.M., n = 7-8 filters per condition). **(D.)** Short-circuit current in mouse colon showing effects of CDCA (1 mM) added to basolateral or apical bathing solutions, with (R)-BPO-27 (10 µM) added as indicated.

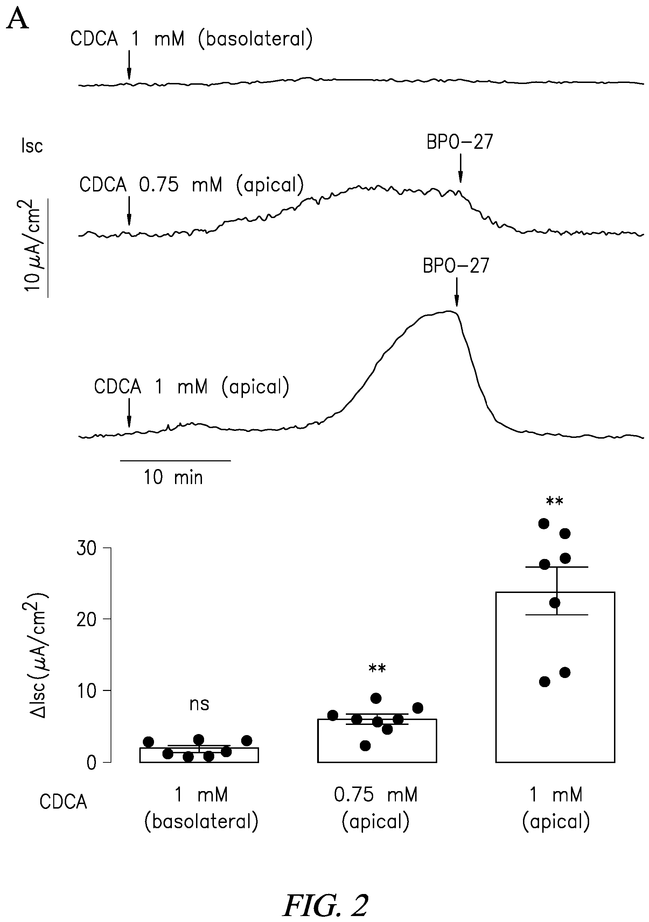

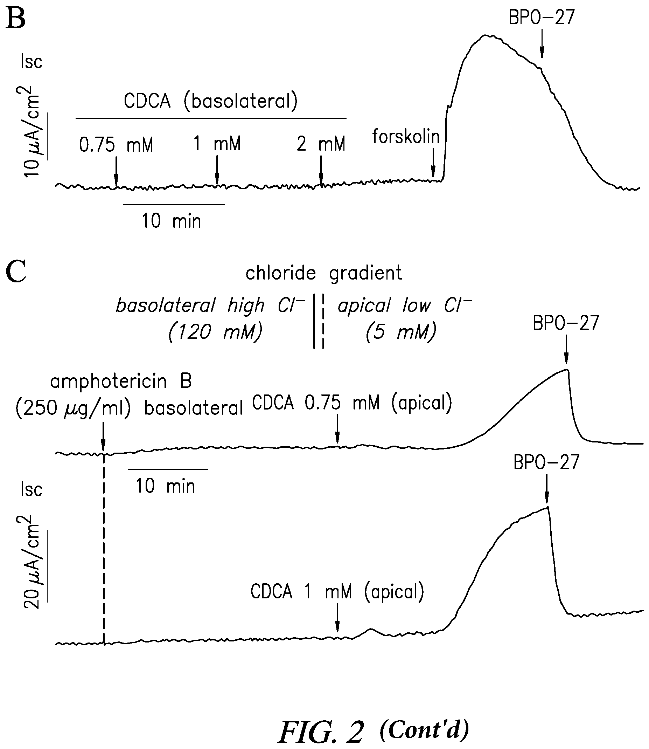

**Figure 2****.** CDCA acts from the apical side in T84 cell monolayers to activate apical CFTR. **(A.)** CDCA is added on the basolateral or apical bathing solutions. (upper) Representative short-circuit current curves, with (R)-BPO-27 added at 10 µM. (lower) Summary of peak CDCA-induced currents (ΔIsc, mean ± S.E.M., n = 7-8, ** p < 0.01, ns, not significant compared to zero effect). **(B.)** Increasing CDCA from 0.75 to 2 mM is added to basolateral bathing solution, followed by forskolin (10 µM added to both sides) and (R)-BPO-27 (10 µM). **(C.)** Short-circuit current in T84 cells following basolateral membrane permeabilization with 250 µg/ml amphotericin B in the presence of a basolateral-to-apical solution Cl⁻ gradient (basolateral [Cl⁻] 120 mM, apical [Cl⁻] 5 mM). CDCA (0.75 or 1 mM) is added to apical bathing solution, followed by (R)-BPO-27 (10 µM).

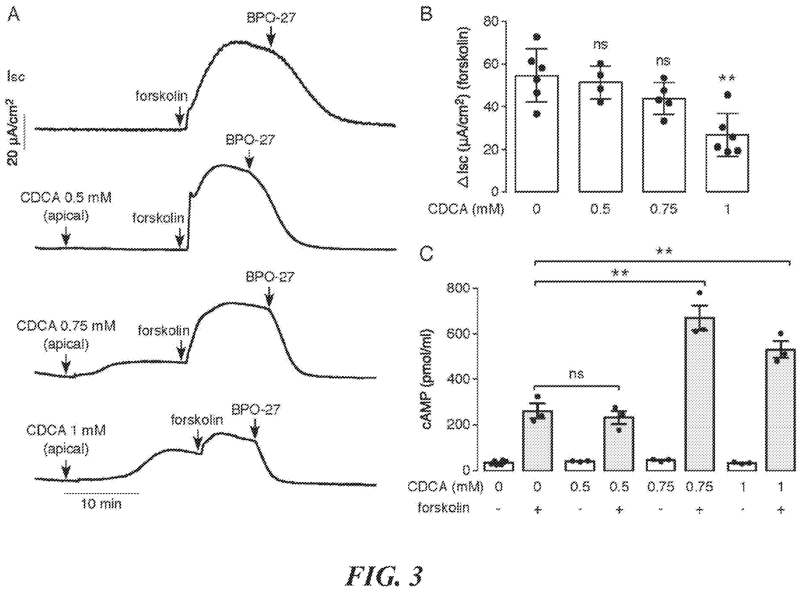

**Figure 3****.** CDCA has minimal effect on cAMP signaling in T84 cells. **(A.)** Short-circuit current showing effects of indicated concentration of CDCA followed by forskolin (10 µM) and then (R)-BPO-27 (10 µM). **(B.)** Summary of peak forskolin-induced current (ΔIsc, mean ± S.E.M., n = 4-6, ** p < 0.01, ns, not significant compared to forskolin response with 0 mM CDCA). **(C.)** cAMP in T84 cells measured 30 min after incubation with indicated concentration of CDCA with or without 10 µM forskolin (mean ± S.E.M., n = 3-6, ** p < 0.01, ns, not significant).

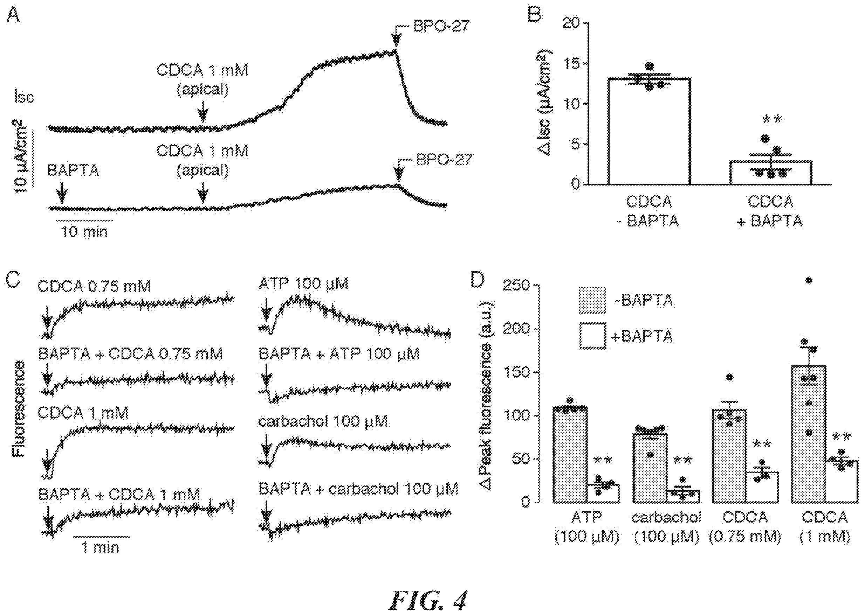

**Figure 4****.** CDCA action in T84 cells involves Ca²⁺ signaling. **(A.)** Short-circuit current showing effect of CDCA (1 mM) after BAPTA-AM (30 µM) pretreatment for 25 min, followed by (R)-BPO-27 (10 µM). **(B.)** Summary of peak CDCA-induced current with or without BAPTA-AM pretreatment (ΔIsc, mean ± S.E.M., n = 4-5, ** p < 0.01). **(C.)** Cytoplasmic Ca²⁺ concentration measured by Fluo-4 fluorescence. CDCA (0.75, 1 mM), ATP (100 µM) or carbachol (100 µM) was added to the apical bathing solution with or without BAPTA-AM (30 µM) pretreatment for 30 min. **(D.)** Peak increase in Fluo-4 fluorescence (mean ± S.E.M., n = 3-6, ** p < 0.01, ns, not significant).

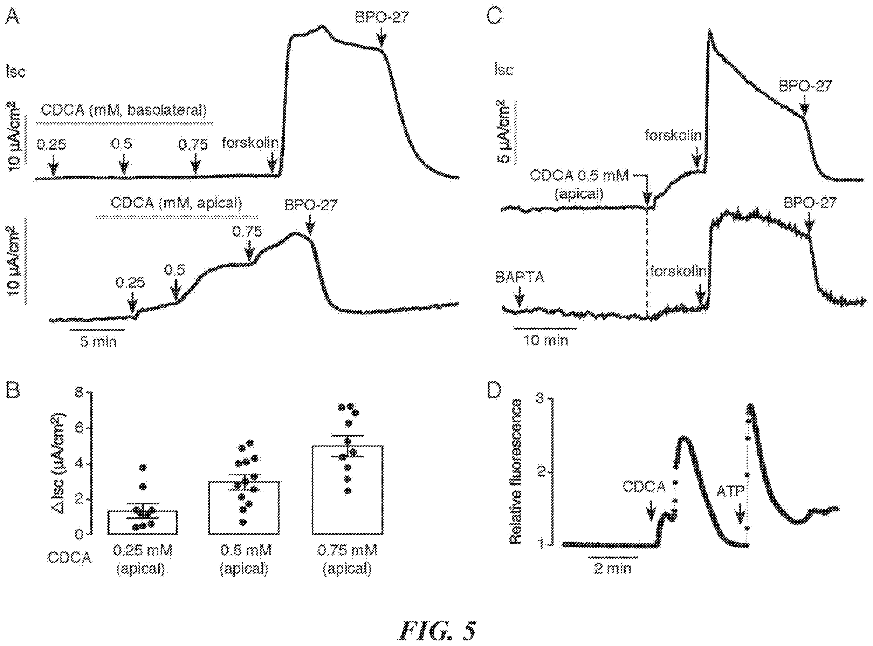

**Figure 5****.** CDCA secretory response in primary cultures of human colonic epithelial cells consisting of planar monolayer cultures generated from colonoids. **(A.)** (upper) Short-circuit current showing effects of indicated CDCA concentration added to the apical or basolateral bathing solutions, followed by forskolin (10 µM) and (R)-BPO-27 (5 µM). **(B.)** Summary of peak CDCA-induced currents (ΔIsc, mean ± S.E.M., n = 9-13). **(C.)** Short-circuit current showing effect of CDCA (0.5 mM) after BAPTA-AM (30 µM) pretreatment for 20 min, followed by forskolin (10 µM) and (R)-BPO-27 (5 µM). **(D.)** Fluorescence measurement of cytoplasmic Ca²⁺ concentration with CDCA (0.75 mM) or ATP (100 µM) added.

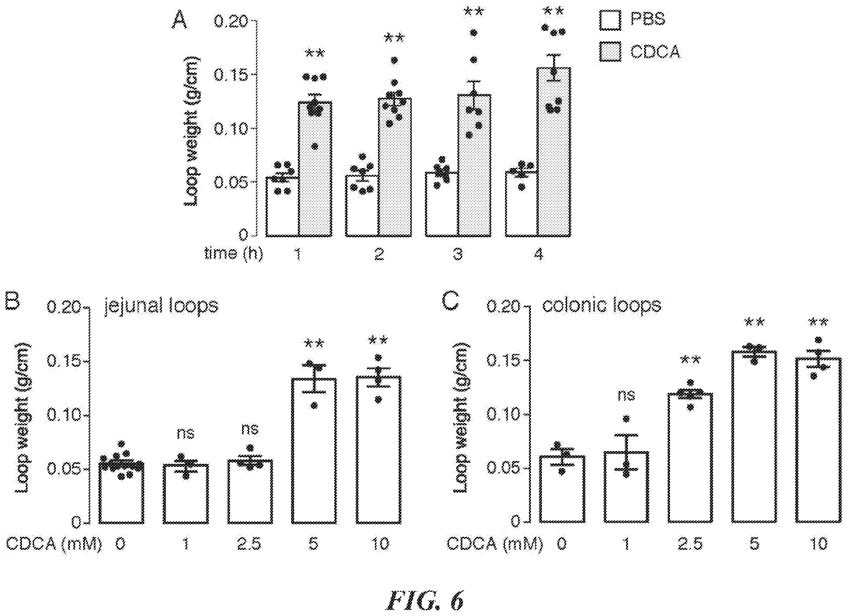

**Figure 6****.** CDCA-induced fluid secretion in closed intestinal loops in mice. **(A.)** Time course of CDCA-induced fluid secretion. 100 µl CDCA in PBS (10 mM) or PBS was injected to mid-jejunal closed loops and loops were excised at different times for measurement **of loop** weight and length (mean ± S.E.M., n = 5-9 loops per group, ** p < 0.01). **(B.)** Concentration-dependence of CDCA effect in closed jejunal loops. 100 µl CDCA or PBS is injected to closed mid-jejunal loops (mean ± S.E.M., n = 3-14 loops per group, ** p < 0.01, ns, not significant). **(C.)** Concentration-dependence of CDCA effect in closed colonic loops). 100 µl CDCA or PBS is injected to closed colonic loops (mean ± S.E.M., n = 3-5 loops per group, ** p < 0.01, ns, not significant).

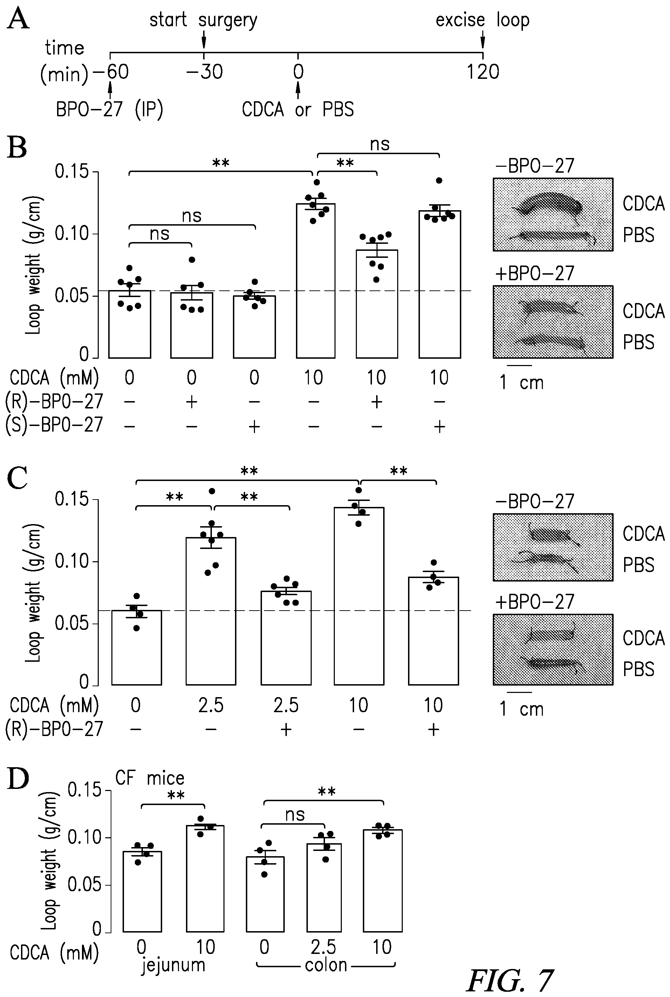

**Figure 7****.** (R)-BPO-27 inhibits CDCA-induced fluid secretion in closed intestinal loops in mice. **(A.)** Experimental protocol. **(B.)** (left) Weight/length ratio of mid-jejunal loops injected with CDCA (10 mM) or PBS with or without BPO-27 (R or S enantiomers) (mean ± S.E.M., n = 6-7 loops per group, ** p < 0.01, ns, not significant). (right) Representative photos of CDCA and PBS injected loops with or without BPO-27. **(C.)** (left) Weight/length ratio of colonic loops injected with CDCA (2.5, 10 mM) or PBS with or without (R)-BPO-27 (mean ± S.E.M., n = 4-7 loops per group, ** p < 0.01, ns, not significant). (right) Representative photos of CDCA and PBS injected loops with or without (R)-BPO-27. **(D.)** Weight/length ratio of mid-jejunal or colonic loops from cystic fibrosis (CFTR-deficient) mice injected with CDCA (2.5, 10 mM) (mean ± S.E.M., n = 4 loops per group, ** p < 0.01, ns, not significant).

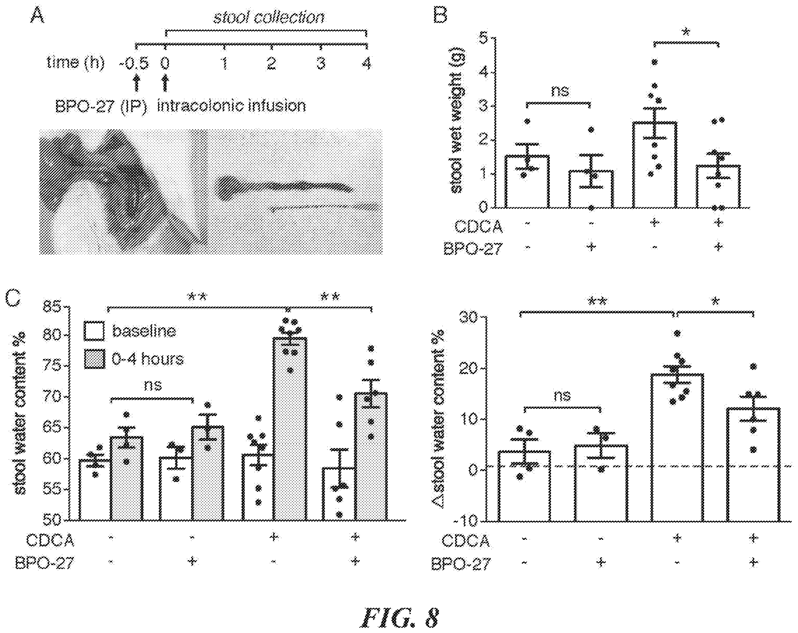

**Figure 8****.** (R)-BPO-27 reduces the increase in stool water content in a rat model of bile acid diarrhea. **(A.)** Experimental protocol (upper) and photos (lower) taken 10 min after mid-colonic infusion of 500 µL of PBS containing Evan's blue dye. **(B.)** Four-hour stool output (wet weight) following mid-colonic infusion in rats with or without (R)-BPO-27 pretreatment (mean ± S.E.M., n = 4 rats in PBS treated group, n = 8 rats in CDCA treated group, * p < 0.05, ns, not significant). **(C.)** (left) Stool water content (percentage water from wet/dry weight measurement) from stool collected in B (** p < 0.01, ns, not significant). (right) Paired analysis showing increase in stool water content of individual rats (stool water at 0-4 hours minus stool water prior to infusion in the same rats) (* p < 0.05, ** p < 0.01, ns, not significant).

### DETAILED DESCRIPTION OF ILLUSTRATIVE EMBODIMENTS

The present invention may be understood by reference to the following detailed description which forms a part of this disclosure.

Scientific and technical terms used in connection with the present application shall have the meanings that are commonly understood by those of ordinary skill in the art, unless otherwise defined herein.

As used herein, the terms "composition," "compound," "drug," "pharmacologically active agent," "active agent," or "medicament" are used interchangeably herein to refer to a compound or compounds or composition of matter which, when administered to a subject (human or mammal) induces a desired pharmacological and/or physiologic effect by local and/or systemic action.

As used herein, the terms "treatment" or "therapy" (as well as different forms thereof) include preventative (*e.g.*, prophylactic), curative, or palliative treatment. As used herein, the term "treating" includes alleviating or reducing at least one adverse or negative effect or symptom of a condition, disease or disorder.

The term "administering" means either directly administering a compound or composition of the present invention.

The term "subject," is used hereinto refer to a human being, or a domesticated mammal (e.g., dog, cat) to whom treatment, including prophylactic treatment, with the compounds according to the present invention, is provided.

Within the present invention, the disclosed compounds may be prepared in the form of pharmaceutically acceptable salts. "Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. These pharmaceutically acceptable salts are prepared by methods known in the art, *e.g.*, by dissolving the free amine bases with an excess of the acid in aqueous alcohol, or neutralizing a free carboxylic acid with an alkali metal base such as a hydroxide, or with an amine.

The present invention is directed to methods of treating a subject having bile acid diarrhea, comprising administering to the subject an amount of a CFTR chloride channel inhibitor, i.e., BPO-27, effective to treat the bile acid diarrhea.

In some aspects, the present invention is directed to methods of treating a subject having bile acid diarrhea. Bile acid diarrhea (BAD) is a clinical diagnosed condition in which diarrhea is thought to result from bile acid malabsorption or disregulation. *See, e.g.,* M. Camilleri, Advances in understanding of bile acid diarrhea, Expert Rev Gastroenterol Hepatol. 2014 January ; 8(1): 49-61. Methods of diagnosing BAD are known in the art. *See, e.g., id.*

In some aspects of the methods disclosed herein, the subject is administered an amount of a CFTR chloride channel inhibitor (CFTR-CCI). As used herein, a CFTR chloride channel refers to the Cystic Fibrosis Transmembrane Conductance Regulator (CFTR), a cAMP-activated chloride channel expressed in epithelia in the lung, intestine, pancreas, testis and other tissues. The term CFTR chloride channel inhibitor (or "CFTR-CCI") as used herein refers to any of the cystic fibrosis transmembrane-conductance regulator chloride channel inhibitors described in the prior art as useful for one or more medicinal purposes, including those described in U.S. Patent Nos. 9,062,073, 7,235,573, 7,638,543, 7,414,037 and 7,888,332; U.S. Application Publication No. 2009/0253799; International Patent Application Publication Nos. WO 09/120803 and WO 09/146144; and Ma T, et al. Thiazolidinone CFTR inhibitor identified by high-throughput screening blocks cholera toxin-induced intestinal fluid secretion. J. Clin. Invest. 2002;110:1651-1658; Sonawane N, Verkman AS. Thiazolidinone CFTR inhibitors with improved water solubility identified by structure-activity analysis. Bioorg. Med. Chem. 2008;16:8187-8195, and Muanprasat C, et al. Discovery of glycine hydrazide pore-occluding CFTR inhibitors: mechanism, structure-activity analysis, and in vivo efficacy. J. Gen. Physiol. 2004;124:125-137.

In some embodiments, not forming part of the present invention, the CFTR chloride channel inhibitor is a BPO CFTR chloride channel inhibitor (BPO-CFTR-CCI). BPO-CFTR-CCIs are compounds encompassed by the generic structure 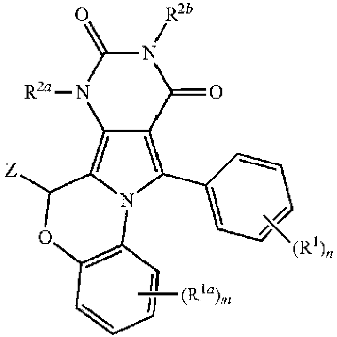 wherein:
m is 1, 2, 3, or 4;
n is 1, 2, 3, 4 or 5;
p is an integer from 0 to 4;
q is an integer from 1 to 4;
R¹ at each occurrence is the same or different and independently H, halo, haloalkyl, C₁-C₆ alkyl, -(CH₂)ₚ-C(O)-R^{4a}, -S(O)₂R^{4a}, -NO₂, or tetrazolyl;
R^{1a} at each occurrence is the same or different and independently H, halo, haloalkyl, C₁-C₆ alkyl, -(CH₂)ₚ-C(O)-R ^{4a}, -S(O)₂R^{4a}, -NO₂, or tetrazolyl;
R^{2a} and R^{2b} are each the same or different and independently H, or C₁-C₆ alkyl;
R^{4a} is -OR⁷, -NR⁷R⁸ -O(CH₂)_{q-}OC(O)R⁷, or an amino acid residue;
R⁷ and R⁸ are each the same or different and independently H, C₁-C₂₀ alkyl, a saccharide, or an amino acid residue; and
Z is aryl or heteroaryl,
wherein the amino acid residue is selected from residues of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, phosphoserine, phosphothreonine, phosphotyrosine, 4-hydroxyproline, hydroxylysine, demosine, isodemosine, gamma-carboxy glutamate, hippuric acid, octahydroindole-2-carboxylic acid, statine, 1,2,3,4-tetrahydroisoquinoline-3-carboxylic acid, penicillamine, omithine, 3-methylhistidine, norvaline, beta-alanine, gamma-aminobutylic acid, cirtulline, homocysteine, homoserine, methyl-alanine, para-benzoylphenylalanine, phenylglycine, propargylglycine, sarcosine, methionine sulfone, tert-butylglycine, 3,5-dibromotyrosine, 3,5-diiodotyrosine, glycosylated threonine, glyclosylated serine, and glycosylated asparagine.

In some embodiments, not forming part of the present invention, the BPO-CFTR-CCI is a compound of formula 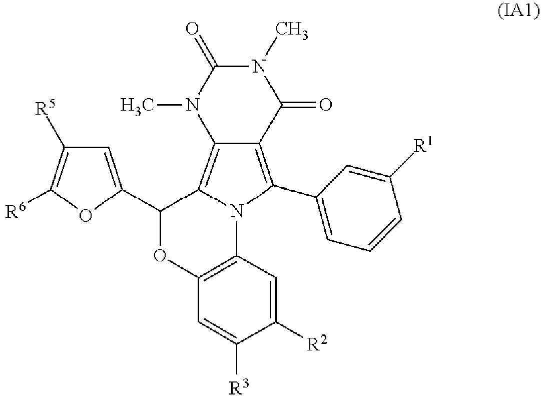 wherein:
R¹ is H, halo, or C₁₋₆ alkyl;
R² and R³ are each the same or different and independently H, halo, -NO₂, C₁₋₆ alkyl, tetrazolyl, -S(O)₂OR⁷, or -C(=O)OR⁷;
R⁵ is H, halo, or C₁₋₆ alkyl;
R⁶ is halo, C₁₋₆ alkyl, or C₁₋₆ haloalkyl; and
R⁷ is H, C₁₋₆ alkyl, a saccharide, an amino acid residue, or a peptide.

BPO-CFTR-CCIs are described in U.S. Patent No. 9,062,073.

In some embodiments, the BPO-CFTR-CCI of the present invention is (R)-BPO-27, a compound having the structure:

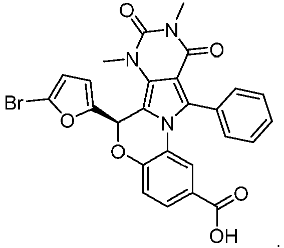

In other embodiments, not forming part of the present invention, the CFTR chloride channel inhibitor is a PPQ CFTR chloride channel inhibitor (PPQ-CFTR-CCI). PPQ-CFTR-CCIs are compounds encompassed by the generic structure 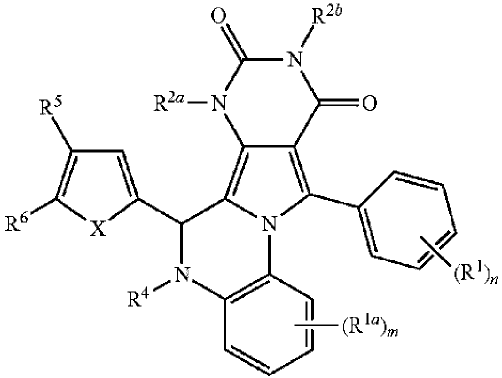 wherein:
m is 1, 2, 3, or 4;
n is 1, 2, 3, 4 or 5;
p is an integer from 0 to 4;
q is an integer from 1 to 4;
X is O or S;
R¹ at each occurrence is the same or different and independently H, halo, haloalkyl, C₁₋₆ alkyl, -(CH₂)ₚ-C(O)-R ^{4a}, -S(O)₂R^{4a}, -NO₂, or tetrazolyl;
R^{1a} at each occurrence is the same or different and independently H, halo, haloalkyl, C₁₋₆ alkyl, -(CH₂)ₚ-C(O)-R^{4a}, -S(O)₂R^{4a}, -NO₂, or tetrazolyl;
R^{2a} and R^{2b} are each the same or different and independently H or C₁₋₆ alkyl;
R^{4a} is -OR⁷, -NR⁷R⁸ -O(CH₂)_{q-}OC(O)R⁷, an amino acid residue, or a peptide;
R⁴ is H, -N(=O), C₁₋₆ alkyl, or haloalkyl;
R⁵ is H, halo, or C₁₋₆ alkyl;
R⁶ is halo, C₁₋₆ alkyl, or C₁₋₆ haloalkyl; and
R⁷ and R⁸ are each the same or different and independently H, C₁₋₂₀ alkyl, a saccharide, an amino acid residue, or a peptide.

PPQ-CFTR-CCIs are described in described in U.S. Patent No. 9,062,073.

In some embodiments, not forming part of the present invention, the CFTR chloride channel inhibitor is a thiazolidinone CFTR chloride channel inhibitor (TD-CFTR-CCI). TD-CFTR-CCIs are compounds encompassed by the generic structure: 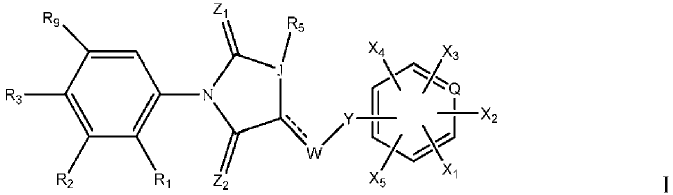 or a pharmaceutically acceptable salt, prodrug, or stereoisomer thereof,
wherein
Y is -NH- or absent;
W is =CH-, -S-, -O-, -C(=S)-, or -C(=O)-;
Z₁, Z₂, Z₃, Z₄, and Z₅ are each independently O or S;
J is C, S, O, or N;
Q is C or N;
R₁, R₂, R₃, and R₉ are each independently H, C₁₋₆ alkyl, alkoxy, halo, -CF₃, -CF₂CF₃, or -OCF₃;
R₅ is H, halo, C₁₋₆ alkyl, or absent;
X₁, X₂, X₃, and X₄ are each independently H, -OH, -SH, halo, tetrazolo, -P(=O)(OH)₂, -C(=Z₃)Z₄H, -Z₅-C(=Z₃) Z₄H, or -Z₅-CH₂-C(=Z₃)Z₄H; and
X₅ is -O⁻, tetrazolo, -C(=O)OH, -O-C(=O)OH, or absent.

TD-CFTR-CCIs are also compounds encompassed by the generic structure: 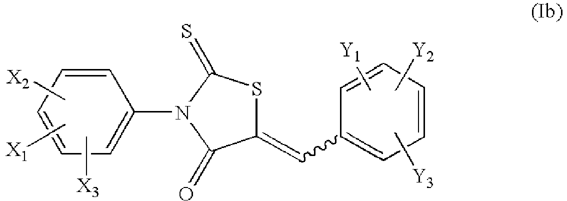
wherein X₁ is trifluoromethyl;
X₂ and X₃ are independently chosen from hydrogen and a halo group;
Y₁, Y₂ and Y₃ are independently chosen from hydrogen, C₁-C₈ alkyl, C₁-C₇ alkoxy, carbonate, carbamate, carboxyl, a halo group, a nitro group, an azo group, a hydroxyl group and a mercapto group.

TD-CFTR-CCIs are also the compounds described in Ma T, et al. Thiazolidinone CFTR inhibitor identified by high-throughput screening blocks cholera toxin-induced intestinal fluid secretion. J. Clin. Invest. 2002;110:1651-1658; and Sonawane N, Verkman AS. Thiazolidinone CFTR inhibitors with improved water solubility identified by structure-activity analysis. Bioorg. Med. Chem. 2008;16:8187-8195; U.S. Patent Nos. 7,235,573, 7,638,543, and International Patent Application Publication No. WO 09/120803.

In some embodiments, not forming part of the present invention, the TD-CFTR-CCI is CFTRᵢₙₕ-172, which has the structure:

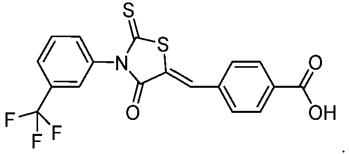

In some embodiments, not forming part of the present invention, the CFTR chloride channel inhibitor is a glycine hydrazide CFTR chloride channel inhibitor (GH-CFTR-CCI). GH-CFTR-CCIs are compounds encompassed by the structure: 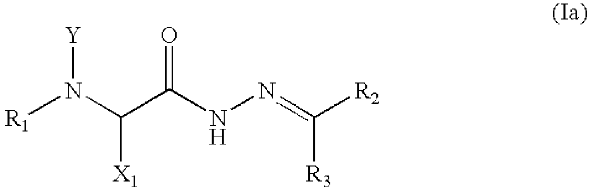 or a pharmaceutically acceptable salt or stereoisomer thereof,
wherein X₁ is hydrogen or a substituted or unsubstituted, saturated linear or branched alkyl;
Y is hydrogen or substituted or unsubstituted, saturated linear or branched alkyl;
R₁ is unsubstituted phenyl,
substituted phenyl wherein phenyl is substituted with one or more of hydroxy, alkyl, and halogen,
substituted or unsubstituted quinolinyl,
substituted or unsubstituted anthracenyl, or
substituted or unsubstituted naphthalenyl;
R² is unsubstituted phenyl,
substituted phenyl, wherein phenyl is substituted with bromo or carboxy,
di(hydroxy)phenyl,
mono-(halo)-mono(hydroxy)phenyl,
mono(halo)-di(hydroxy)phenyl,
mono(halo)-tri(hydroxy)phenyl,
di(halo)-mono(hydroxy)phenyl,
di(halo)-di(hydroxy)phenyl,
di(halo)-tri(hydroxy)phenyl,
mono(halo)-mono(hydroxy)-mono(alkoxy)phenyl,
mono(halo)-di(hydroxy)-mono(alkoxy)phenyl,
mono(halo)-mono(hydroxy)-di(alkoxy)phenyl,
mono(halo)-di(hydroxy)-di(alkoxy)phenyl,
di(halo)-mono(hydroxy)-mono(alkoxy)phenyl,
di(halo)-di(hydroxy)-mono(alkoxy)phenyl,
di(halo)-mono(hydroxy)-di(alkoxy)phenyl; and
R₃ is hydrogen or substituted or unsubstituted alkyl.

GH-CFTR-CCIs are also compounds encompassed by the generic formula: 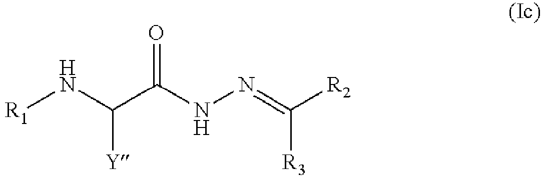 or a pharmaceutically acceptable salt or stereoisomer thereof,
wherein Y" is a substituted or unsubstituted, saturated linear or branched alkyl; or an amide or ether linker attached to a polar molecule, wherein the polar molecule is selected from a substituted or unsubstituted phenyl group, a polyoxyalkyl polyether, a polyethyleneimine, a disaccharide, a trisaccharide, a polyalkylimine, and a small amino dextran;
R₁ is unsubstituted or substituted phenyl, substituted or unsubstituted quinolinyl, substituted or unsubstituted anthracenyl, or substituted or unsubstituted naphthalenyl; R² is unsubstituted or substituted phenyl; and
R₃ is hydrogen or substituted or unsubstituted alkyl, thereby inhibiting CFTR

GH-CFTR-CCIs are also compounds encompassed by the generic formula: 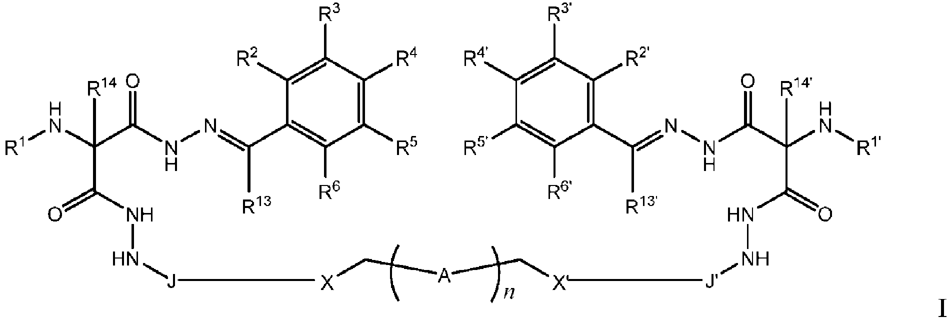 or a pharmaceutically acceptable salt, prodrug, or stereoisomer thereof
wherein:
R¹ and R¹ are the same or different and independently optionally substituted phenyl, optionally substituted heteroaryl, optionally substituted quinolinyl, optionally substituted anthracenyl, or optionally substituted naphthalenyl;
R², R^{2'}, R³, R^{3'}, R⁴, R^{4'}, R⁵, R^{5'}, R⁶, and R^{6'} are each the same or different and independently hydrogen, hydroxy, Ci g alkyl, Ci g alkoxy, carboxy, halo, nitro, cyano, -SO3H, -S(=O)2NH2, aryl, and heteroaryl;
R¹³, R¹³ , R¹⁴, and R¹⁴ are each the same or different and independently hydrogen or C₁₋₈ alkyl;
X and X' are each the same or different linker moiety;
J and J' are each the same or different spacer moiety;
A is a polymer subunit; and n is an integer between 0 and 2,500.

GH-CFTR-CCIs are also the compounds described in Muanprasat C, et al. Discovery of glycine hydrazide pore-occluding CFTR inhibitors: mechanism, structure-activity analysis, and in vivo efficacy. J. Gen. Physiol. 2004;124:125-137; U.S. Patent Nos. 7,414,037 and 7,888,332; U.S. Application Publication No. 2009/0253799; International Patent Application Publication No. WO 09/146144.

In some embodiments, not forming part of the present invention, the GH-CFTR-CCI is GlyH-101, which has the structure

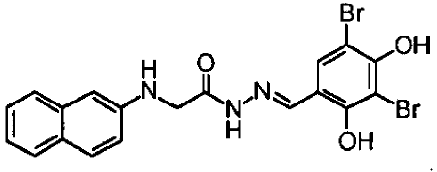

Other CFTR chloride channel inhibitors not forming part of the present invention are glibenclamide, diphenylamine-2-carboxylate, 5-nitro-2-(3-phenylpropylamino) benzoate, and niflumic acid.

In some embodiments of the methods disclosed herein, but not forming part of the present invention, the subject is administered a CFTR chloride channel inhibitor that is a BPO-CFTR-CCI, a PPQ-CFTR-CCI, a TD-CFTR-CCI, or a GH-CFTR-CCI.

In some embodiments of the methods disclosed herein, the subject is administered (R)-BPO-27.

In some embodiments of the methods of the disclosure, the subject is administered a pharmaceutical composition comprising a pharmaceutical excipient and an amount of a CFTR chloride channel inhibitor, i.e., BPO-27.

In some aspects, the disclosure is directed to methods of treating a subject having bile acid diarrhea, comprising administering to the subject a pharmaceutical composition comprising an amount of a CFTR chloride channel inhibitor, i.e., BPO-27 effective to treat the bile acid diarrhea. In some embodiments, the BPO-CFTR-CCI is (R)-BPO-27. In some embodiments, the pharmaceutical composition further comprises (S)-BPO-27.

In those embodiments wherein the subject is administered a pharmaceutical composition comprising a CFTR chloride channel inhibitor that is (R)-BPO-27, the pharmaceutical composition may also include (S)-BPO-27. In these embodiments, the amount of (R)-BPO-27 in the pharmaceutical composition will be substantially equal to, or more than, the amount of (S)-BPO-27 present in the pharmaceutical composition. For example, in some embodiments, the pharmaceutical composition may include a racemic mixture of (R/S)-BPO-27. In some aspects, the (R)-BPO-27 will be present in an enantiomeric excess (ee), as compared to the (S)-BPO-27. For example, the (R)-BPO-27 can be present in about a 1, 2, 3, 4, 5, 6, 7, 8, 9, 10, 15, 20, 25, 30, 35, 40, 45, 50, 55, 60, 65, 70, 75, 80, 85, 90, 95, or 99 %ee. In some embodiments, the pharmaceutical composition will include (R)-BPO-27 wherein the %ee is greater than 99%.

The amount of CFTR chloride channel inhibitor administered is an amount that is effective to treat the bile acid diarrhea. The amount that is effective in this regard will vary depending on the subject's characteristics and condition.

In some aspects of the disclosed methods, the amount of CFTR chloride channel inhibitor is effective to reduce intestinal fluid secretion resulting from the bile acid diarrhea.

In other aspects of the disclosed methods, the amount of CFTR chloride channel inhibitor is effective to reduce bile acid-induced activation of apical CFTR chloride channels.

In some aspects of the disclosed methods, subject is also administered an amount of a second agent effective to treat the bile acid diarrhea. In some embodiments, the second agent is a bile acid binder, a farnesoid X receptor (FXR) agonist, a 5-HT3 antagonist, an opioid receptor agonist, a mixed µ opioid receptor agonist, a broad-spectrum gut-specific antibiotic, an antispasmodic, or a tricyclic antidepressant. In some embodiments, the second agent is a bile acid binder, preferably cholestyramine, colestipol, or colesevelam. In other embodiments, the second agent is a farnesoid X receptor (FXR) agonist, preferably obeticholic acid. In other embodiments, the second agent is a 5-HT3 antagonist, preferably alosetron. In other embodiments, the second agent is an opioid receptor agonist, preferably loperamide. In other embodiments, the second agent is a mixed µ opioid receptor agonist, preferably eluxadoline. In other embodiments, the second agent is a broad-spectrum gut-specific antibiotic, preferably rifaximin.

In some aspects, the present invention is directed to methods of reducing intestinal fluid secretion resulting from bile acid-induced activation of CFTR chloride channels in the intestinal epithelium in a subject in need thereof, comprising administering to the subject an amount of a CFTR chloride channel inhibitor, i.e., BPO-27, effective to reduce the intestinal fluid secretion resulting from bile acid-induced activation of CFTR chloride channels.

As used herein, intestinal fluid secretion refers to secretion of fluid from the intestinal epithelia into the intestinal lumen.

In some embodiments, the intestinal fluid secretion results from bile acid-induced activation of CFTR chloride channels in the intestinal epithelium. In other embodiments, the CFTR chloride channels are apical CFTR chloride channels in the intestinal epithelium. Apical CFTR chloride channels are CFTR chloride channels located in the apical cell membrane (i.e., the cell membrane facing the intestinal lumen). Thus, in some embodiments, the present invention is directed to methods of reducing intestinal fluid secretion resulting from bile acid-induced activation of apical CFTR chloride channels in the intestinal epithelium in a subject in need thereof, comprising administering to the subject an amount of a CFTR chloride channel inhibitor effective to reduce the intestinal fluid secretion resulting from bile acid-induced activation of apical CFTR chloride channels.

In some aspects, the intestinal fluid secretion is reduced by administering an amount of a CFTR chloride channel inhibitor, i.e., BPO-27, effective to reduce said intestinal fluid secretion. As used herein, reduction of intestinal fluid secretion refers to a decrease in the amount of fluid that is secreted into the intestinal lumen relative to the amount of fluid that is secreted into the intestinal lumen in the absence of administration of a CFTR chloride channel inhibitor. Methods of measuring decrease in fluid secretion are known to those skilled in the art, and include measuring the water content of the intestinal luminal contents, and diarrhea output.

In some embodiments, the intestinal fluid secretion is reduced by administering a CFTR chloride channel inhibitor that is (R)-BPO-27, In some embodiments, the CFTR chloride channel inhibitor is (R)-BPO-27.

In this aspect of the invention, the amount of CFTR chloride channel inhibitor administered is an amount that is effective to reduce said intestinal fluid secretion resulting from bile acid-induced activation of CFTR chloride channels (e.g., the apical CFTR chloride channels). The amount that is effective in this regard will vary depending on the subject's characteristics and condition.

In some aspects, the present invention is directed to methods of reducing intestinal fluid secretion resulting from bile acid-induced activation of CFTR chloride channels in the intestinal epithelium in a subject in need thereof, comprising administering to the subject a pharmaceutical composition comprising an amount of a CFTR chloride channel inhibitor, i.e., BPO-27, effective to reduce the intestinal fluid secretion resulting from bile acid-induced activation of CFTR chloride channels. In some embodiments, the CFTR chloride channel inhibitor is a BPO-CFTR-CCI, wherein the BPO-CFTR-CCI is (R)-BPO-27. In some embodiments, the pharmaceutical composition further comprises (S)-BPO-27.

In other aspects, the disclosure is directed to methods of reducing bile acid-induced CFTR chloride channel current in the intestinal epithelium of a subject in need thereof, comprising administering to the subject an amount of a CFTR chloride channel inhibitor, i.e., BPO-27, effective to reduce said bile acid-induced CFTR chloride channel current.

As used here, "current" refers to the passage of ions through the channel. Thus, CFTR chloride channel current refers to the passage of chloride ions through the CFTR chloride channel.

In some embodiments, the CFTR chloride channel current results from bile acid-induced activation of apical CFTR chloride channels in the intestinal epithelium. Thus, in some embodiments, the present invention is directed to methods of reducing bile acid-induced apical CFTR chloride channel current in the intestinal epithelium of a subject in need thereof, comprising administering to the subject an amount of a CFTR chloride channel inhibitor effective to reduce said bile acid-induced apical CFTR chloride channel current.

In some embodiments, the bile acid-induced CFTR chloride channel current in the intestinal epithelium is reduced by administering a CFTR chloride channel inhibitor that is (R)-BPO-27.

In some embodiments, the CFTR chloride channel inhibitor is (R)-BPO-27.

In this aspect of the invention, the amount of CFTR chloride channel inhibitor administered is an amount that is effective to reduce the bile acid-induced CFTR chloride channel (e.g., the apical CFTR chloride channels) current. The amount that is effective in this regard will vary depending on the subject's characteristics and condition.

In some aspects, the present invention is directed to methods of reducing bile acid-induced CFTR chloride channel current in the intestinal epithelium of a subject in need thereof, comprising administering to the subject a pharmaceutical composition comprising an amount of a CFTR chloride channel inhibitor effective to reduce said bile acid-induced CFTR chloride channel current. In some embodiments, the CFTR chloride channel inhibitor is a BPO-CFTR-CCI, wherein the BPO-CFTR-CCI is (R)-BPO-27. In some embodiments, the pharmaceutical composition further comprises (S)-BPO-27.

In all of the methods of the disclosure, the CFTR chloride channel inhibitor is administered to a subject. In some embodiments, the subject is a mammal. In other embodiments, the subject is a human.

In some embodiments, the subject has been diagnosed with Crohn's disease.

In other embodiments, the subject has been diagnosed with IBS-D.

In yet other embodiments, the subject has been diagnosed with functional diarrhea.

In some aspects, the methods of the present invention result in a reduction in the water content of the subject's stool. The extent of water reduction can be determined by measuring the amount of water in the subject's stool before administering the CFTR chloride channel inhibitor, and comparing that amount to the amount of water in the subject's stool after administering the CFTR chloride channel inhibitor.

In some embodiments, the reduction in the water content of the subject's stool is demonstrated by measurement using a clinical instrument such as, for example, the Bristol Stool Form Scale (BSFS). The BSFS, which is known in the art, assigns the subject's stool a consistency score ranging from 1 (hard) to 7 (watery). Thus, in some embodiments, the reduction in the water content of the subject's stool using the present methods is demonstrated by a decrease in score using the Bristol Stool Form Scale. In some embodiments, the reduction in the water content of the subject's stool using the present methods is demonstrated by a decrease of 1 point (e.g., 7 to 6, 6 to 5, 5 to 4, 4 to 3, 3 to 2, 2 to 1) using the Bristol Stool Form Scale. In other embodiments, the reduction in the water content of the subject's stool using the present methods is demonstrated by a decrease of 2 points (e.g., 7 to 5, 6 to 4, 5 to 3, 4 to 2, 3 to 1) using the Bristol Stool Form Scale. In other embodiments, the reduction in the water content of the subject's stool using the present methods is demonstrated by a decrease of 3 points (e.g., 7 to 4, 6 to 3, 5 to 2, 4 to 1) using the Bristol Stool Form Scale. In other embodiments, the reduction in the water content of the subject's stool using the present methods is demonstrated by a decrease of more than 3 points using the Bristol Stool Form Scale.

It will be understood by those skilled in the art that the reduction in the water content of the subject's stool can be demonstrated using other clinical scales known the art.

In some aspects, the methods of the present invention result in a reduction in the subject's frequency of defecation. The extent of defecation frequency reduction can be determined by comparing the subject's defecation frequency before administering the CFTR chloride channel inhibitor to the subject's defection frequency after administering the CFTR chloride channel inhibitor. Here, defection frequency may be measured by, for example, interviewing the subject, or administering to the subject an instrument designed to elicit this information. Methods of determining defecation frequency are known to those in the art.

In other aspects, the methods of the present invention result in a reduction in the subject's stool output. The extent of stool output reduction can be determined by measuring the quantity of subject's stool before administering the CFTR chloride channel inhibitor, and comparing that amount to the quantity of the subject's stool after administering the CFTR chloride channel inhibitor. Here, the quantity may be measured by weight or by volume. Methods for measuring stool output are known to those in the art.

In other aspects, the methods of the present invention result in a reduction in the subject's abdominal pain. The extent of abdominal pain reduction can be determined by comparing the subject's abdominal pain before administering the CFTR chloride channel inhibitor with the subject's abdominal pain after administering the CFTR chloride channel inhibitor. Here, abdominal pain may be measured by, for example, interviewing the subject, or administering to the subject an instrument designed to elicit this information. Methods of determining abdominal pain are known to those in the art.

In other aspects, the methods of the present invention result in a reduction in the subject's abdominal bloating. The extent of abdominal bloating reduction can be determined by comparing the subject's abdominal bloating before administering the CFTR chloride channel inhibitor with the subject's abdominal bloating after administering the CFTR chloride channel inhibitor. Here, abdominal bloating may be measured by, for example, interviewing the subject, or administering to the subject an instrument designed to elicit this information. Methods of determining abdominal bloating are known to those in the art.

In other aspects, the methods of the present invention result in a reduction in the subject's nausea. The extent of nausea reduction can be determined by comparing the subject's nausea before administering the CFTR chloride channel inhibitor with the subject's nausea after administering the CFTR chloride channel inhibitor. Here, nausea may be measured by, for example, interviewing the subject, or administering to the subject an instrument designed to elicit this information, such as the Nausea Questionnaire. Methods of measuring nausea are known to those in the art.

### Examples

The following examples are provided to provide a better understanding of the subject matter described herein. These examples should not be considered to limit the described subject matter.

### Abbreviations

BAD: bile acid diarrhea
BAPTA-AM: 1,2-Bis(2-aminophenoxy)ethane-N,N,N',N'-tetraacetic acid tetrakis(acetoxymethyl ester)
BPO-27: 6-(5-Bromofuran-2-yl)-7,9-dimethyl-8,10-dioxo-11-phenyl-7,8,9,10-tetrahydro-6H-benzo[b]pyrimido [4',5':3,4]pyrrolo [1,2-d][1,4]oxazine-2-carboxylic acid
CaCC: calcium-activated chloride channel
CDCA: chenodeoxycholic acid
CFTR: cystic fibrosis transmembrane conductance regulator
CFTRᵢₙₕ-172: 4-[[4-Oxo-2-thioxo-3-[3-(trifluoromethyl)phenyl]-5-thiazolidinylidene]methyl]benzoic acid
DCA: deoxycholic acid
DRA: down-regulated in adenoma
ENaC: epithelial sodium channel
FGF-19: fibroblast growth factor 19
FXR: farnesoid X receptor
GlyH-101: *N*-2-naphthalenyl-[(3,5-dibromo-2,4-dihydroxyphenyl)methylene]glycinehydrazide
IBS-D: diarrhea-predominant irritable bowel syndrome
IC₅₀: half-maximal inhibitory concentration

### Chemicals

Unless specified otherwise, all chemicals were purchased from Sigma-Aldrich (St. Louis, MO). Sodium salts of tauro-chenodeoxycholic acid and tauro-deoxycholic acid were purchased from Spectrum Chemicals (Gardena, CA). All bile acid solutions were prepared in PBS, except for lithocholic acid which was dissolved in DMSO. BAPTA-AM was purchased from EMD Millipore (Billerica, MA). Forskolin was purchased from Lc Laboratories (Woburn, MA). CFTRᵢₙₕ-172 and (R)-BPO-27 were synthesized and purified as described. *See, e.g.,* Snyder, D. S., Tradtrantip, L., Yao, C., Kurth, M. J., and Verkman, A. S. (2011) Potent, metabolically stable benzopyrimido-pyrrolo-oxazine-dione (BPO) CFTR inhibitors for polycystic kidney disease. J Med Chem 54, 5468-5477; Snyder, D. S., Tradtrantip, L., Battula, S., Yao, C., Phuan, P. W., Fettinger, J. C., Kurth, M. J., and Verkman, A. S. (2013) Absolute configuration and biological properties of enantiomers of CFTR inhibitor BPO-27. ACSMed Chem Lett 4, 456-459; Ma, T., Thiagarajah, J. R., Yang, H., Sonawane, N. D., Folli, C., Galietta, L. J., and Verkman, A. S. (2002) Thiazolidinone CFTR inhibitor identified by high-throughput screening blocks cholera toxin-induced intestinal fluid secretion. J Clin Invest 110, 1651-1658.

### Example 1. T84 cell culture

T84 cells (ATCC CCL-248) are cultured in a 1:1 mixture of DMEM/Ham's F-12 medium supplemented with 10% FBS, 100 U/mL penicillin and 100 µg/mL streptomycin. Cells are grown on Snapwell inserts (Costar Coming, Horseheads, NY) at 37 °C in 5% CO₂/95% air and used 7-14 days after plating.

### Example 2. Human colonoid cultures

Human colonoid cultures are generated from de-identified tissue samples (other than age and sex) obtained from endoscopic or surgical procedures from three different subjects.. Human cultures are generated from isolated intestinal crypts embedded in Matrigel (Corning, Tewksbury, MA) in 24-well plates and cultured in the presence of Wnt3A, R-spondin-1 and Noggin containing undifferentiated media (UDM) as described in Zachos, N. C. et. al (2016) Human enteroids/colonoids and intestinal organoids functionally recapitulate normal intestinal physiology and pathophysiology. J Biol Chem 291, 3759-3766; In, J., et al. (2016) Enterohemorrhagic escherichia coli reduce mucus and intermicrovillar bridges in human stem cell-derived colonoids. Cell Mol Gastroenterol Hepatol 2, 48-62. Matrigel cultures in UDM media are used for the propagation of colonoids. To generate monolayer cultures, colonoids are triturated in Cultrex Organoid Harvesting Solution (Trevigen, Gaithersburg, MD), and the fragments are collected by centrifugation and resuspended in UDM. Colonoid fragments (100 µL) are seeded onto 0.4 µm pore polyester membrane 24-well cell culture inserts (Transwell; Corning, Tewksbury, MA) pre-coated with human collagen IV (34 µg/mL; Millipore Sigma). Monolayers are cultured in UDM at 37 °C, 5% CO₂. Under these conditions, cultures reach confluency in 7-14 days as monitored by transepithelial resistance.

### Example 3. Short-circuit current measurements

T84 cells are mounted in Ussing chambers and bathed in symmetrical HCO₃⁻-buffered solution containing (in mM): 120 NaCl, 5 KCl, 1 MgCl₂, 1 CaCl₂, 10 D-glucose, 5 HEPES and 25 NaHCO₃ (pH 7.4). The solutions are aerated with 95% O₂/5% CO₂ and maintained at 37 °C. In one study, for measurement of apical Cl⁻ conductance, a basolateral to apical Cl⁻ gradient is applied in which the basolateral hemichamber contains (in mM): 120 NaCl, 1 MgCl₂, 1 CaCl₂, 10 D-glucose, 5 HEPES and 25 NaHCO₃ (pH 7.4); in the apical solution 120 mM NaCl is replaced by 5 mM NaCl and 115 mM Na-gluconate, and the basolateral membrane is permeabilized with 250 µg/ml amphotericin B. Short-circuit current is measured using an EVC4000 multichannel voltage clamp (World Precision Instruments, Sarasota, FL).

For intestinal short-circuit current measurement, CD1 mice are anesthetized with isoflurane. The colon is removed, washed with ice-cold Krebs buffer, opened along the mesenteric border, and a full-thickness fragment is mounted in a micro-Ussing chamber (area 0.7 cm², World Precision Instruments). Hemichambers are filled with oxygenated Krebs-bicarbonate solution.

Measurements in human colonoid cultures are done as described (18). The apical and basolateral hemichambers are filled with Krebs-Ringer bicarbonate (KBR) buffer that is gassed with 95% O₂/5% CO₂ at 37 °C. The basolateral hemichamber is supplemented with 10 mM glucose and the apical hemichamber is supplemented with 10 mM mannitol to maintain osmotic balance.

### Example 4. Intracellular Ca²⁺ and cAMP measurements

T84 cells are plated in 96-well black-walled microplates. Confluent cells are loaded with Fluo-4 NW (Invitrogen, Carlsbad, CA) at 72 h after plating. For Ca²⁺ measurement Fluo-4 fluorescence is measured with a FluoStar fluorescence plate reader (BMG Lab Technologies, Durham, North Carolina) at excitation/emission wavelengths of 485/538 nm. In some studies cells are pretreated for 30 min with BAPTA-AM. Ca²⁺ measurement in human colonoid cultures is done in monolayers that are transduced with the fluorescent Ca²⁺ sensor Adeno-GCsMP6s. For cAMP assay, T84 cells are grown in 24-well plates, treated for 30 min with CDCA and/or forskolin, lysed by repeating freeze/thaw, centrifuged to remove cell debris, and the supernatant is assayed for cAMP using the Parameter cAMP immunoassay kit according to the manufacturer's instructions (R&D Systems, Minneapolis, NM).

### Example 5. Intestinal fluid secretion in closed intestinal loops in mice

CD1 mice (age 8-10 weeks) are given access to 5% dextrose water but not solid food for 24 h before experiments. Mice are administered (R)-BPO-27 (5 mg/kg) or vehicle (5% DMSO, 10% Kolliphor HS in saline) intraperitoneally 60 min before creation of closed intestinal (mid-jejunal or distal colonic) loops and injection of CDCA or PBS vehicle. This dose of (R)-BPO-27 was previously shown to give therapeutic serum levels for several hours in mice. *See* Cil, O., et al. (2017) Benzopyrimido-pyrrolo-oxazine-dione CFTR inhibitor (R)-BPO-27 for antisecretory therapy of diarrheas caused by bacterial enterotoxins. FASEB J 31, 751-760. For creation of loops, mice are anesthetized with isoflurane, and body temperature is maintained during surgery at 36-38 °C using a heating pad. A small abdominal incision is made to expose the intestine. For the mid-jejunal closed-loop model, 2-3 cm loops are created by sutures. Loops are injected with 100 µl PBS containing CDCA or PBS vehicle. The abdominal incision is closed with sutures, and mice are allowed to recover from anesthesia. In the colonic closed-loop model, mice are given an enema (500 µl of mineral oil) 12 hours before surgery to cleanse the colon of solids as previously reported (Haggie, P. M., et al. (2018) SLC26A3 inhibitor identified in small molecule screen blocks colonic fluid absorption and reduces constipation. JCI Insight 3, e121370) and a 1-2 cm closed loop is created by sutures. Intestinal loops are surgically removed at specified times, and loop length and weight is measured to quantify fluid secretion. In some experiments, CF mice (ΔF508 homozygous, age 8-10 weeks) are used.

### Example 6. Rat model of bile acid diarrhea

Female Sprague-Dawley rats (age 8-10 weeks) are administered 500 µl CDCA (10 mM in PBS) or PBS vehicle by a mid-colonic infusion using a flexible plastic tube (15 gauge, 78 mm length, Instech Laboratories, Plymouth Meeting, PA). Rats are intraperitoneally administered (R)-BPO-27 (10 mg/kg) (or vehicle control) 30 min before infusion. (R)-BPO-27 is dissolved in saline containing 5% DMSO and 10% Kolliphor HS. Following the infusion, rats are placed individually in metabolic cages and given free access to water and food. Stool samples are collected prior to infusion (for baseline stool water content) and over 4 hours after the infusion. Stools are weighed immediately when visualized to determine wet weight. To determine stool water content, the stool samples are dried at 70°C for 24 hours and water content is calculated as (wet weight - dry weight) / wet weight.

### Example 7. Statistics

Data are presented as mean ± S.E.M. Statistical analysis is performed using Prism 5 GraphPad Software package (San Diego, CA). Statistical comparisons are made using the-Student's t-test when there are two groups or one-way analysis of variance when there are three groups or more. A p value of < 0.05 is taken as statistically significantly.

### CFTR inhibition blocks the bile acid-induced secretory response in T84 cells

In initial studies multiple bile acids are screened by short-circuit current measurements for their ability to induce a prosecretory response in the T84 colonic epithelial cell line. *See* Example 3. When added to both the apical (mucosal) and basolateral (serosal) bathing solutions the bile acids CDCA and DCA, and their taurine conjugates, produce a robust secretory current that is blocked by (R)-BPO-27 (Figure 1A). No increase in short-circuit current is seen for cholic acid and ursodeoxycholic acid, and only a small transient response is seen with lithocholic acid.

The increase in short-circuit current following CDCA application is fully blocked by the selective and chemically unrelated inhibitors (R)-BPO-27 and CFTRᵢₙₕ-172 (Figure 1B, C). Concentration-dependence studies show an IC₅₀ for (R)-BPO-27 inhibition in this model of <1 µM (data not shown). Short-circuit current measurements in mouse distal colon show an increase in current of 5.2 ± 1.4 µA/cm² (mean ± S.E.M., n = 3) with CDCA addition to the apical bathing solution, which is fully reversed by (R)-BPO-27 (-5.2 ± 1.7 µA/cm²) (Figure 1D). No increase in short-circuit current is seen following CDCA addition to the basolateral bathing solution.

To investigate the sidedness of CDCA action in T84 cells, CDCA is added to either the apical or basolateral bathing solutions. Figure 2A shows a robust current response upon addition of 0.75 or 1 mM CDCA to the apical solution, without effect of 1 mM CDCA added to the basolateral solution. Little effect is seen of up to 2 mM CDCA added to the basolateral solution (Figure 2B), with forskolin added at the end of the study as a positive control to demonstrate integrity of the T84 cell monolayer. CDCA action on apical CFTR and inhibition by (R)-BPO-27 is confirmed in short-circuit current measurements in basolateral membrane-permeabilized T84 cell monolayers in the presence of an apical-to-basolateral Cl⁻concentration gradient, in which short-circuit current provides a direct measure of apical CFTR Cl⁻ current (Figure 2C).

Possible CDCA actions on cAMP and Ca²⁺ signaling pathways in T84 cells are investigated. *See* Example 4. CDCA up to 0.75 mM does not significantly affect the short-circuit response to addition of the cAMP agonist forskolin, but a small reduction in forskolin-induced current is seen with 1 mM CDCA (Figure 3A, B). In each case the increased current was fully blocked by (R)-BPO-27. Intracellular cAMP measurement shows that CDCA up to 1 mM does not increase cAMP by itself. However, 0.75 and 1 mM CDCA increases cAMP in response to maximal (10 µM) forskolin (Figure 3C).

The CDCA-induced increase in short-circuit current in T84 cells is largely blocked by pretreatment with the Ca²⁺ chelator BAPTA-AM (Figure 4A, B). Measurements of intracellular Ca²⁺ concentration using the fluorescent sensor Fluo-4 show a sustained Ca²⁺ elevation following 0.75 or 1 mM CDCA, with comparable magnitude to the peak Ca²⁺ elevation seen with the cholinergic agonist carbachol or the purinergic agonist ATP (Figure 4C, D). The Ca²⁺ elevations are largely blocked by BAPTA-AM pretreatment. These experiments support the involvement of Ca²⁺ signaling in the prosecretory response to CDCA in T84 cells, which was unexpected because the secretory response appears to be fully mediated by CFTR, a cAMP-activated Cl⁻ channel, and not by Ca²⁺-activated Cl⁻ channels.

### Studies in primary human colonoid cultures

The major findings obtained using T84 cells, including the CFTR Cl⁻ secretory response to apical CDCA, and the involvement Ca²⁺ signaling, are investigated in primary human colonoid cultures. *See* Example 2. As seen with T84 cells, CDCA produces a concentration-dependent increase in short-circuit current when added to the apical but not basolateral bathing solution, which is reversed by (R)-BPO-27 (Figure 5A, B). Also, the increase in short-circuit current is greatly reduced by BAPTA-AM pretreatment (Figure 5C), and CDCA produces an elevation in cytoplasmic Ca²⁺ concentration with peak magnitude comparable to that produced by ATP (Figure 5D), albeit more transient than that seen in T84 cells.

### BPO-27 inhibits fluid secretion in closed murine intestinal loops

A closed-intestinal loop model is established in mice to investigate the prosecretory action of CDCA in vivo. *See* Example 5. Initial studies are done in mid-jejunal loops because of the technical ease of obtaining multiple loops in a single animal and their extensive prior use in studying intestinal fluid secretion in models of cholera and Traveler's diarrhea. Figure 6A shows that injection of 10 mM CDCA in mid-jejunal loops produces robust accumulation of fluid by 1 hour. A CDCA concentration-dependence study with loop fluid measured at 2 hours shows a significant increase in loop fluid accumulation with 5 and 10 mM CDCA (Figure 6B). Similar experiments in closed distal colonic loops show significant loop fluid accumulation with 2.5 mM CDCA (Figure 6C).

To study effects of CFTR inhibition, (R)-BPO-27 is administered 60 min prior to creation of closed intestinal loops and injection of CDCA (or control PBS vehicle) (Figure 7A). In closed mid-jejunal loops, the active BPO-27 enantiomer, (R)-BPO-27 produces a significant, 55% reduction in loop fluid accumulation, whereas inactive enantiomer (S)-BPO-27 has no significant effect (Figure 7B). Greater reductions in loop fluid accumulation with (R)-BPO-27 of ~70% was found in closed colonic loops (Figure 7C).

Since CFTR inhibition largely but not completely blocks loop fluid accumulation, whether CFTR-independent mechanisms might in part be responsible from CDCA effect is investigated. For these experiments closed-loop studies are done in cystic fibrosis mice lacking functional CFTR. Mid-jejunal and distal colonic closed loops injected with 10 mM CDCA show a small but significantly greater loop fluid content compared to control loops injected with PBS vehicle, suggesting mechanisms other than CFTR also playing a role in CDCA-induced fluid accumulation, which based on prior studies might include inhibition of Na⁺/H⁺ and Cl⁻/HCO₃⁻ exchangers (pro-absorptive processes), increased paracellular permeability, or stimulation of mucus secretion. *See* Alrefai, W. A., et al. (2007) Taurodeoxycholate modulates apical Cl-/OH- exchange activity in Caco2 cells. Dig Dis Sci 52, 1270-1278; Pallagi-Kunstar, E., et al. (2015) Bile acids inhibit Na+/H+ exchanger and Cl-/HCO3- exchanger activities via cellular energy breakdown and Ca2+ overload in human colonic crypts. Pflugers Arch 467, 1277-1290; Sarathy, J., et al. (2017) The yin and yang of bile acid action on tight junctions in a model colonic epithelium. Physiol Rep 5, e13294; Barcelo, A., et al. (2001) Effect of bile salts on colonic mucus secretion in isolated vascularly perfused rat colon. Dig Dis Sci 46, 1223-1231

### BPO-27 reduces stool water content in a rat model of bile acid diarrhea

A model is established to test (R)-BPO-27 effect in which CDCA (or vehicle control) is administered to rats by a mid-colonic infusion, with stool collected over 4 hours for measurement of stool weight and water content (by wet-to-dry weight ratio) (Figure 8A, upper). *See* Example 6. Infused fluid in this model rapidly distributes through the colonic lumen as seen using Evans blue dye (Figure 8A, lower). A greater amount of stool is collected over 4 hours from rats infused with CDCA, and stool water content is significantly increased by ~25 % (Figure 8B, C). While (R)-BPO-27 pretreatment does not affect stool quantity or water content in rats that receive a mid-colonic infusion of PBS, the increase in stool water content in rats receiving CDCA infusion is ~55 % inhibited (Figure 8C).

Without being bound by theory, the data herein support of the utility of CFTR inhibition by CFTR inhibitors, particularly (R)-BPO-27, in the treatment of bile acid-associated diarrheas. Short-circuit current measurements in T84 cells and human colonoid cultures show a prosecretory current in response to exposure of the cell apical surface to CDCA, which is fully reversed by CFTR inhibition. Complete inhibition of secretory current by (R)-BPO-27 is also seen in CDCA-exposed mouse colonic tissue ex vivo. Systemically administered (R)-BPO-27 largely prevents fluid accumulation in closed colonic loops in mice in vivo, and prevents the increase in stool water following intracolonic infusion of a CDCA-containing solution. These results implicate CFTR as a major prosecretory ion channel in CDCA-induced diarrhea in human colonic cell cultures and rodents.

This application claims the benefit of priority to U.S. Provisional Application No. 62/860,539 filed June 12, 2019.

## Claims

1. BPO-27, or a pharmaceutical composition comprising BPO-27, for use in a method of treating a subject having bile acid diarrhea.

2. BPO-27, or a pharmaceutical composition comprising BPO-27 for use of claim 1, wherein the BPO-27 is (R)-BPO-27.

3. BPO-27, or a pharmaceutical composition comprising BPO-27 for use of claim 2, wherein the pharmaceutical composition further comprises (S)-BPO-27.

4. BPO-27, or a pharmaceutical composition comprising BPO-27 for use of claim 3, wherein the pharmaceutical composition includes a racemic mixture of (R/S)-BPO-27

5. The BPO-27, or a pharmaceutical composition comprising BPO-27 for use of any one of the preceding claims, wherein the BPO-27 is effective to reduce intestinal fluid secretion resulting from the bile acid diarrhea.

6. BPO-27, or a pharmaceutical composition comprising BPO-27 for use of any one of the preceding claims, being further combined with a second agent effective to treat the bile acid diarrhea, wherein said second agent is a bile acid binder, preferably cholestyramine, colestipol, or colesevelam, a farnesoid X receptor (FXR) agonist, preferably obeticholic acid, a 5-HT3 antagonist, preferably alosetron, an opioid receptor agonist, preferably loperamide, a mixed µ opioid receptor agonist, preferably eluxadoline, a broad-spectrum gut-specific antibiotic, preferably rifaximin, an antispasmodic, or a tricyclic antidepressant.

7. BPO-27, or a pharmaceutical composition comprising BPO-27 for use of any one of the preceding claims, wherein the subject is a human.

8. BPO-27, or a pharmaceutical composition comprising BPO-27 for use of any one of the preceding claims, wherein the subject (1) has been diagnosed with Crohn's disease; (2) has been diagnosed with IBS-D; or (3) has functional diarrhea.

9. BPO-27, or a pharmaceutical composition comprising BPO-27 for use of any one of the preceding claims, wherein said use results in (1) a reduction in the water content of the subject's stool; (2) reduction in the subject's frequency of defecation; (3) reduction in the subject's stool output; (4) reduction in the subject's abdominal pain; (5) reduction in the subject's abdominal bloating; or (6) reduction in the subject's nausea.

10. BPO-27, or a pharmaceutical composition comprising BPO-27, for use of claim 9, wherein said reduction in the water content of the subject's stool is demonstrated by a decrease in in the subject's score on the Bristol Stool Form Scale.

## Patentansprüche

1. BPO-27 oder pharmazeutische Zusammensetzung, die BPO-27 umfasst, zur Verwendung in einem Verfahren zur Behandlung eines Subjekts, das eine Gallensäurediarrhö aufweist.

2. BPO-27 oder pharmazeutische Zusammensetzung, die BPO-27 umfasst, zur Verwendung nach Anspruch 1, wobei das BPO-27 (R)-BPO-27 ist.

3. BPO-27 oder pharmazeutische Zusammensetzung, die BPO-27 umfasst, zur Verwendung nach Anspruch 2, wobei die pharmazeutische Zusammensetzung ferner (S)-BPO-27 umfasst.

4. BPO-27 oder pharmazeutische Zusammensetzung, die BPO-27 umfasst, zur Verwendung nach Anspruch 3, wobei die pharmazeutische Zusammensetzung ein racemisches Gemisch von (R/S)-BPO-27 enthält.

5. BPO-27 oder pharmazeutische Zusammensetzung, die BPO-27 umfasst, zur Verwendung nach einem der vorhergehenden Ansprüche, wobei das BPO-27 wirksam ist, um die aus der Gallensäurediarrhö resultierende intestinale Flüssigkeitssekretion zu verringern.

6. BPO-27 oder pharmazeutische Zusammensetzung, die BPO-27 umfasst, zur Verwendung nach einem der vorhergehenden Ansprüche, ferner kombiniert mit einem zweiten Mittel, das wirksam ist, um die Gallensäurediarrhö zu behandeln, wobei das zweite Mittel ein Gallensäurebindemittel, vorzugsweise Cholestyramin, Colestipol oder Colesevelam, ein Farnesoid-X-Rezeptor(FXR)-Agonist, vorzugsweise Obeticholsäure, ein 5-HT3-Antagonist, vorzugsweise Alosetron, ein Opioidrezeptor-Agonist, vorzugsweise Loperamid, ein gemischter µ-Opioidrezeptor-Agonist, vorzugsweise Eluxadolin, ein Darm-spezifisches Breitspektrum-Antibiotikum, vorzugsweise Rifaximin, ein Antispasmodikum oder ein trizyklisches Antidepressivum ist.

7. BPO-27 oder pharmazeutische Zusammensetzung, die BPO-27 umfasst, zur Verwendung nach einem der vorhergehenden Ansprüche, wobei das Subjekt ein Mensch ist.

8. BPO-27 oder pharmazeutische Zusammensetzung, die BPO-27 umfasst, zur Verwendung nach einem der vorhergehenden Ansprüche, wobei bei dem Subjekt (1) Morbus Crohn diagnostiziert wurde; (2) RDS-D diagnostiziert wurde; oder (3) funktionelle Diarrhö vorliegt.

9. BPO-27 oder pharmazeutische Zusammensetzung, die BPO-27 umfasst, zur Verwendung nach einem der vorhergehenden Ansprüche, wobei die Verwendung zu (1) einer Verringerung des Wassergehalts des Stuhls des Subjekts; (2) einer Verringerung der Defäkationshäufigkeit des Subjekts; (3) einer Verringerung der Stuhlausscheidung des Subjekts; (4) einer Verringerung der abdominalen Schmerzen des Subjekts; (5) einer Verringerung der abdominalen Blähungen des Subjekts; oder (6) einer Verringerung der Übelkeit des Subjekts führt.

10. BPO-27 oder pharmazeutische Zusammensetzung, die BPO-27 umfasst, zur Verwendung nach Anspruch 9, wobei die Verringerung des Wassergehalts des Stuhls des Subjekts durch eine Abnahme des Werts des Subjekts auf der Bristol-Stuhlformen-Skala nachgewiesen wird.

## Revendications

1. BPO-27, ou composition pharmaceutique comprenant du BPO-27, destiné(e) à être utilisé(e) dans un procédé de traitement d'un sujet souffrant de diarrhée liée aux acides biliaires.

2. BPO-27, ou composition pharmaceutique comprenant du BPO-27 destiné(e) à être utilisé(e) selon la revendication 1, ledit BPO-27 étant le (R)-BPO-27.

3. BPO-27, ou composition pharmaceutique comprenant du BPO-27 destiné(e) à être utilisé(e) selon la revendication 2, ladite composition pharmaceutique comprenant en outre le (S)-BPO-27.

4. BPO-27, ou composition pharmaceutique comprenant du BPO-27 destiné(e) à être utilisé(e) selon la revendication 3, ladite composition pharmaceutique comprenant un mélange racémique de (R/S)-BPO-27.

5. BPO-27, ou composition pharmaceutique comprenant du BPO-27 destiné(e) à être utilisé(e) selon l'une quelconque des revendications précédentes, ledit BPO-27 étant efficace pour réduire la sécrétion de fluide intestinal résultant de la diarrhée liée aux acides biliaires.

6. BPO-27, ou composition pharmaceutique comprenant du BPO-27 destiné(e) à être utilisé(e) selon l'une quelconque des revendications précédentes, étant en outre combiné(e) avec un second agent efficace pour traiter la diarrhée liée aux acides biliaires, ledit second agent étant un liant des acides biliaires, de préférence la cholestyramine, le colestipol ou le colésévélam, un agoniste du récepteur farnésoïde X (FXR), de préférence l'acide obéticholique, un antagoniste 5-HT3, de préférence l'alosétron, un agoniste du récepteur opioïde, de préférence le lopéramide, un agoniste mixte du récepteur opioïde µ, de préférence l'eluxadoline, un antibiotique à large spectre spécifique de l'intestin, de préférence la rifaximine, un antispasmodique ou un antidépresseur tricyclique.

7. BPO-27, ou composition pharmaceutique comprenant du BPO-27 destiné(e) à être utilisé(e) selon l'une quelconque des revendications précédentes, ledit sujet étant un être humain.

8. BPO-27, ou composition pharmaceutique comprenant du BPO-27 destiné(e) à être utilisé(e) selon l'une quelconque des revendications précédentes, ledit sujet (1) ayant reçu un diagnostic de maladie de Crohn ; (2) ayant reçu un diagnostic de syndrome de l'intestin irritable à prédominance diarrhéique (IBS-D) ; ou (3) présentant une diarrhée fonctionnelle.

9. BPO-27, ou composition pharmaceutique comprenant du BPO-27 destiné(e) à être utilisé(e) selon l'une quelconque des revendications précédentes, ladite utilisation entraînant (1) une réduction de la teneur en eau des selles du sujet ; (2) une réduction de la fréquence de défécation du sujet ; (3) une réduction de la quantité de selles du sujet ; (4) une réduction des douleurs abdominales du sujet ; (5) une réduction des ballonnements abdominaux du sujet ; ou (6) une réduction des nausées du sujet.

10. BPO-27, ou composition pharmaceutique comprenant du BPO-27, destiné(e) à être utilisé(e) selon la revendication 9, ladite réduction de la teneur en eau des selles du sujet étant démontrée par une diminution du score du sujet sur l'échelle de Bristol.
